# EUROPEAN PATENT APPLICATION

(11) **EP 1 374 703 A1**
(43) Date of publication of application: **02.01.2004**
(21) Application number: 03013959.6
(22) Date of filing: 20.06.2003
(51) Int. Cl.: A23L 1/305, A23J 1/04, A23J 3/04, A23L 1/30, A61K 35/48, A61K 35/54, A61K 9/19, A61K 7/48

(54) **Use of a non-human animal embryo extract for the preparation of a nutritive substance and a dietetic-nutraceutical supplement**

(30) Priority: 20.06.2002 IT MI20021362
(71) Applicant: Biava, Pier Mario, 20123 Milano (IT); Ricotti, Annamaria, 20123 Milano (IT)
(72) Inventor: Biava, Pier Mario, 20123 Milan (IT)
(74) Representative: Gervasi, Gemma, Dr.

(57) **Abstract**

Use of an animal embryo extract for preparing nutritive compositions for oral administration for general nutrition or for topical application for nutrition of the skin and cutaneous appendages.

## Description

### Prior Art

The use of animal embryo homogenates and extracts for the treatment of tumorous diseases is known.

For example in French patent A-2451193 the use of a homogenate consisting of a hydroalcoholic extract of cow, sheep or swine embryos for the treatment of tumours is described.

In patent application WO 98/11905 a composition suitable for the treatment of tumours containing an embryo or gravid uterus extract is described in which the embryos are removed from oviparous or ovoviviparous animals and the gravid uteri are removed from mice, rabbit, sheep or swine.

European patent application N. 97928188.8 reports that embryo extracts removed at specific stages of embryonic development are effective for the treatment of tumours and other pathologies controlled by the anti-oncogene p 53 and that said specific stages change dependent upon the species of origin of the extracts.

### Summary

We have now found that extracts of animal embryos, removed at appropriate stages of embryonic development, are very effective when administered to man as nutritive substances.

Administration can be oral for general nutrition or topical for nutrition of the skin and cutaneous appendages. In particular, oral administration as a dietetic-nutraceutical supplement is very effective for energy recovery in debilitated persons, while topical application is very effective as an anti-ageing treatment.

### Detailed description of the invention

The characteristics and advantages of using an animal embryo extract as nutritive substance according to the present invention will be illustrated more fully in the following detailed description.

Said embryo extract is formulated in compositions suitable for oral administration and topical application, in mixture with pharmaceutically acceptable diluents or excipients.

The present invention therefore also regards compositions comprising an effective quantity of animal embryo extract to be used as a nutrient suitable for oral administration or topical application.

Embryos suitable for use according to the present invention are removed from fishes, birds or amphibians. Preferred fishes are trout and brachydanio rerio, preferred birds are chickens and quails and preferred amphibians are frogs.

The relative embryos are removed at the stages of embryonic development corresponding to:
midblastula-gastrula, 5-somites and 20-somites.

A method suitable for obtaining animal embryo extracts is described hereinafter. The embryos are washed with distilled water and then suspended in a solution consisting of 85% pure glycerol and 15% ethyl alcohol solution 30% in water. In said solution, the embryos at the various stages of embryonic development are suspended in the following weight ratio: 66% at the midblastula-gastrula stage, 17% at the 5-somites stage, and 17% at the 20-somites stage. The suspension is treated with two cycles of ultrasounds for 10 seconds and is then treated in a turboemulsifier for 3 minutes. The suspension obtained is diluted with purified water, containing from 400 to 2000 mg/l grapefruit extracts, in a volume ratio of 1:10 and is then filtered under vacuum with a 90 micrometre Millipore membrane and subsequently with a 10 micrometre membrane. A solution is thus obtained having a total concentration of proteins derived from the embryos of about 3 µg/ml. The solution thus obtained can be lyophilised and used for preparing compositions in the form of solutions, tablets and capsules for oral administration and lotions, gels, ointments and creams for topical application.

The lyophilised extract has a protein content between 80 and 90%. Said compositions comprise proteins extracted from embryos according to the invention in mixture with solvents or excipients suitable for use as nutritive compositions.

Oral administration as a dietetic-nutraceutical supplement is very effective in persons debilitated by age or by chronic or degenerative diseases.

The effect of the extracts according to the present invention involves, in such cases, a substantial recovery of energy and weight together with an improvement in performances.

Topical application provides nutrition for the skin and cutaneous appendages with anti-ageing and anti-alopecia effects.

The doses of extract for oral administration are between 9 and 30 µg of extracted proteins /day.

For topical anti-ageing treatment ointments are preferred, containing between 5 and 20% of embryo extract to be applied twice a day.

For topical anti-alopecia treatment lotions are preferred, containing between 5 and 20% of embryo extract to be applied twice a day.

Some preparations of compositions according to the present invention are reported hereinafter in which EPEP indicates the brachydanio rerio embryo extract obtained according to the present invention

### Example 1

### Preparation of a composition in tablet form

A solution of EPEP containing 3 µg/ml of protein is lyophilised together with mannitol. The lyophilised product is mixed with Polycarbophyl (Noveon A1®), Mg stearate and Avicel pH 101®. The mixture is sifted with an 0.8 mm sieve and then compressed to hence prepare tablets weighing 75 mg/tablet and with a 7 mm diameter.

The amounts of the various ingredients used in the preparation are the following:

| | |
|---|---|
| EPEP | 0.250 mg |
| Mg stearate | 7.0 " |
| Polycarbophyl (Noveon®) | 10.0 " |
| (Goodrich-Milan) | |
| Mannitol | 30.0 " |
| Avicel pH 101® (Eigenmann and Veronelli Milan) | remainder to 75.0 " |

### Example 2

### Preparation of a composition in cream form

a) Xalofin®, Nesatol®, glycerin and Euxil® are melted together in a container temperature controlled at 70°C.
b) Water is heated separately to 80°C.
c) The mixture from stage a) is added to the water from stage b) and the suspension obtained is cooled under vigorous agitation.
d) When the suspension from stage c) reaches 30°C, EPEP is added, the suspension is cooled to 25°C and perfume is added.

The amounts by weight of the various substances used in the preparation are the following:

| | |
|---|---|
| EPEP | 13.7% |
| Xalifin 15®(VEVY-Genoa) | 10.0% |
| Nesatol® (VEVY-Genoa) | 9.0% |
| Glycerin | 3.0% |
| Euxil K 400 (Schulke-Majr-Milan) | 0.2% |
| Perfume | 0.1% |
| Purified water | remainder to 100% |

### Example 3

### Preparation of a composition in gel form

a) EPEP is diluted with distilled water, phosphatidylcholine is added and the mixture is agitated using a turbine to produce a liposomal dispersion.
b) Glycerin is added under agitation to the dispersion from stage a) then the thickening agent Carbopol® is added.
c) Agitation is continued until the Carbopol® is completely hydrated.

The amounts by weight of the various substances used in the preparation are as follows:

| | |
|---|---|
| EPEP | 13.7% |
| Ethyl alcohol | 10.0% |
| Phosphatidylcholine | 0.5% |
| Glycerin | 3.0% |
| Carbopol 940® (Goodrich Milan) | 1.0% |
| Purified water | remainder to 100% |

### Example 4

### Preparation of a composition in trichological lotion form

Distilled water, ethyl alcohol, EPEP, glycerol and panthenol are loaded into a dissolver equipped with an agitator.

The solution obtained is filtered through a 0.45 micrometre membrane.

The amounts by weight of the various substances used in the preparation are as follows:

| | |
|---|---|
| EPEP | 13.7% |
| Ethyl alcohol | 20.0% |
| Glycerol | 3.0% |
| Panthenol | 0.1% |
| Purified water | remainder to 100% |

### Application trials of the compositions of the invention

The effectiveness of the use of animal embryo extracts of the present invention as nutritive substances is demonstrated by the application trials which follow.

### A) Clinical trials for the study of systemic effects

In these trials 50 subjects were involved (25 males and 25 females) aged between 60 and 80 years. 30 of the subjects were affected by advanced stage neoplasia of various types, whose traditional therapies of well-established effectiveness had been abandoned and the subjects were thus being treated only with palliative cures. 20 of them were not affected by any particular pathologies but were in poor nutritional condition.

To these subjects dosages of brachydanio rerio extract were administered prepared as abovementioned, in quantities between 9 and 30 ug of protein /day in 3-10 ml of solution divided into three administrations.

The clinical trial was conducted in the following manner:
1) evaluation of body weight and its variations;
2) evaluation of performance status using the ECOG scale (which comprises the following grades: 0=patient fully active; 1=patient showing symptoms but almost completely ambulatory; 2=patient confined to bed for less than 50% of the time; 3=patient confined to bed for more than 50% of the time; 4=patient completely bed-ridden). Administration of the embryo extracts was carried out for 2 consecutive months at the aforestated daily doses.

The results of said trial are as follows:
A) In the 30 patients with neoplastic disease, the performance status, which was for all at grade 3 prior to treatment, passed to grade 2 in 9 patients and to grade 1 in 18 patients, while remaining at grade 3 in 3 patients (a very significant improvement equivalent to 90% of patients treated).
   Such an improvement appeared in general after three weeks of treatment and was maintained as such after 2 months from the start thereof. An average body weight gain of 1.3 Kg (S.D.=0.4) was achieved.
B) In the subjects without particular pathologies and in poor nutritional condition, body weight gain was 1.8 Kg (S.D.=0.6) at the end of 2 months. The grade 1 performance status prior to treatment remained the same. In addition to appetite improvement and body weight increase all subjects also reported a subjective sense of improved well-being.

### B) Evaluation of localised effect on the skin

The extract of brachydanio rerio embryo prepared in the aforestated manner was incorporated in cosmetic cream or gel as described in the abovementioned examples (examples 2 and 3).

The clinical trials concerned 18 subjects with very dry skin conditions and with deep wrinkles both on the face and the hands. The treatment consisted of applying the aforesaid cosmetic cream twice a day for 2 months.

The results of the clinical trials have indicated an increase in skin hydration with substantial reduction in wrinkle depth and smoothing of small wrinkles in 15 of the 18 subjects (an 83% improvement). In addition, a reduced yellowing of the skin, symptomatic of ageing, was noticed in these 15 subjects, together with an increased translucency and luminosity of the skin.

The nutritive products for oral administration by sublingual absorption comprise drops, prepared as in the aforestated method, and also mucoadhesive dietetic tablets, prepared as in example 1. The products for topical administration comprise cream (example 2) and gel (example 3). For improving the nutritional condition of the scalp the production of a trichological lotion is proposed (example 4).

## Claims

1. Use of an animal embryo extract with a protein content between 80 and 90% of the dry weight for the preparation of compositions with nutritive function.

2. Use as claimed in claim 1, **characterised in that** said embryo extract is an extract of fish or bird or amphibian embryos.

3. Use as claimed in claim 1, **characterised in that** said embryo extract is an extract of animal embryos chosen from the group consisting of trout, brachydanio rerio, chicken, quail and frog.

4. Use as claimed in claim 1, **characterised in that** said embryo extract is an extract of embryos removed at the midblastula-gastrula, 5-somites and 20-somites stages.

5. Use as claimed in claim 1, **characterised in that** said nutritive function comprises general nutrition and nutrition of the skin and cutaneous appendages.

6. Compositions comprising an effective quantity of an animal embryo extract with a protein content between 80 and 90% of the dry weight mixed with acceptable diluents or excipients, suitable for administration as nutrients.

7. Compositions as claimed in claim 6, **characterised in that** the preparation is in the form of solutions for oral administration as dietetic-nutraceutical supplements.

8. Compositions as claimed in claim 6, **characterised in that** the preparation is in the form of tablets or capsules for oral administration as dietetic-nutraceutical supplements.

9. Compositions as claimed in claim 6, **characterised in that** the preparation is in the form of lotions, gels, ointments or creams for topical application.

10. Method suitable for supplementing nutrition comprising administration of an animal embryo extract with a protein content between 80 and 90% of the dry weight.

11. Method as claimed in claim 10, **characterised in that** a quantity of extracted proteins between 9 and 30 µg/day is administered orally.

12. Method as claimed in claim 10, **characterised in that** ointments or lotions containing from 5 to 20% of embryo extract are applied topically twice a day.

13. Process for preparing animal embryo extract with a protein content between 80 and 90% of the dry weight comprising the following stages:
a) Fish or bird or amphibian embryos, removed at the embryonic development stages of midblastula-gastrula, 5-somites and 20-somites, are washed with distilled water and then suspended in a solution consisting of 85% glycerol and 15% ethyl alcohol solution 30% in water;
b) the suspension obtained from stage a) is treated with two cycles of ultrasound for 10 seconds and then treated in a turboemulsifier for 3 minutes;
c) the suspension obtained from stage b) is diluted in a volume ratio of 1:10 with purified water containing from 400 to 2000 mg/l of grapefruit extract;
d) the suspension obtained from stage c) is filtered under vacuum with a 90 micrometre Millipore membrane and subsequently with a 10 micrometre membrane;
e) the solution obtained from stage d) is lyophilised.

14. Process as claimed in claim 13, **characterised in that** said embryos used in stage a) have a weight ratio of: 66% embryos removed at the midblastula-gastrula stage, 17% embryos removed at the 5-somites stage and 17% embryos removed at the 20-somites stage.
